# EUROPEAN PATENT SPECIFICATION

(11) **EP 2 393 453 B1**
(45) Date of publication and mention of the grant of the patent: **04.07.2012**
(21) Application number: 10705188.0
(22) Date of filing: 08.02.2010
(51) Int. Cl.: A61F 2/42

(54) **EXPANDABLE JOINT IMPLANT**
EXPANDIERBARES GELENKIMPLANTAT
IMPLANT ARTICULAIRE DILATABLE

(30) Priority: 06.02.2009 US 202211 P
(43) Date of publication of application: 14.12.2011
(73) Proprietor: Ortho-Space Ltd., 44643 Kfar-Saba (IL)
(72) Inventor: SHOHAT, Shaul, 73134 Kfar HaOranim (IL)
(74) Representative: KIPA AB
(86) International application number: PCT/IB2010/050562
(87) International publication number: WO 2010/097724

(56) References cited:
- WO-A1-98/56317
- WO-A1-02/054992
- WO-A1-2004/093767
- US-A- 4 467 479

## Description

### FIELD AND BACKGROUND OF THE INVENTION

The present invention, in some embodiments thereof, relates to body implantable devices and, more particularly, but not exclusively, to a small bones implant device for implanting between small bones.

A human thumb metacarpal is connected to the wrist through a first carpometacarpal (CMC) joint also known as the trapeziometacarpal joint. A base of the thumb metacarpal articulates with a saddle shaped trapezium, the saddle shaped articulation providing a stability required for grasping actions. Furthermore, the saddle shaped articulation allows for thumb motion in a tri-axial mode, which may make the joint relatively susceptible to arthritic degeneration. Soft tissue surrounding the joint, including the beak ligament, may additionally contribute to the arthritic degeneration by causing joint surface subluxation. Damage to the CMC joint may therefore be debilitating and may require surgical intervention when other measures do not suffice.

Patients suffering from arthritis of the CMC joint may recur to surgical intervention as a possible solution to their problem. A commonly employed solution is CMC joint arthroplasty also referred to as trapezectomy. This may be performed by an open surgical procedure or by an arthroscopic procedure, a goal of the surgical intervention being to obtain a painless strong thumb without losing motion or causing deformation.

A number of reconstructive procedures are known in the art for treating CMC joint arthritis. These include interpositional arthroplasty, resection arthroplasty of the trapezium, resection interpositional arthroplasty of the trapezium, total and partial joint replacement arthroplasty of the CMC joint (several types of prosthetic joints), and arthroscopic procedures such as CMC arthroplasty and CMC joint arthrodesis.

US Patent No. 7,037,342 "IMPLANT FOR RECONSTRUCTIO OF JOINTS" relates to "A spacer member (1) is intended to be placed between the ends of the bones which are to be connected, one end of the spacer member being designed to form a joint surface against one of the bone ends (6,7). A joint-stabilizing connection (2,3) is arranged to connect the bones. The spacer member (1) is made of at least one tissue-compatible material."

US Publication No. 2006/0241778 "INTERPOSITIONAL BIARTICULAR DISK" relates to "An interpositional biarticular disk implant (11) having a circular peripheral rim, a generally toroidal axial center opening (13) and convex upper and lower surfaces (15, 17) is implanted between resected concave surfaces of the metacarpal base and the trapezium or other carpal bone in a CMC joint replacement. The disk (11) is anchored in operative position through the use of a flexable cond, such as a harvested tendon that passes through the center opening (13) and through osseous passageways created in the two facing bones."

The closest prior art is disclosed in WO 98/56317.

### SUMMARY OF THE INVENTION

There is provided in accordance with an exemplary embodiment of the invention, a soft, expandable, implantable device sized for spacing between small bones comprising a first smooth surface on which a first small bone may slide.

In an exemplary embodiment of the invention, said device is biodegradable in the body. Optionally or alternatively, said small bones comprise bones of a human hand. Optionally or alternatively, said small bones comprise bones of a human foot.

In an exemplary embodiment of the invention, said small bones comprise at least one of a trapezium, a trapezoid bone, a metacarpal bone, and a scaphoid bone.

In an exemplary embodiment of the invention, the device comprises an inflation port for inflating said device.

In an exemplary embodiment of the invention, the device comprises at least one passage extending from a distal side to a proximal side of the device for promoting fibrotic development between said small bones.

In an exemplary embodiment of the invention, the device comprises at least one passage extending from a first lateral side to an opposite second lateral side of the device for promoting fibrotic development between said small bones. Optionally or alternatively, at least one of said at least one passage includes has a diameter that is at least 20% of a maximal extent of the device.

In an exemplary embodiment of the invention, the device comprises a mesh for promoting fibrotic development between said small bones. Optionally, the mesh covers a portion of an external surface of said device. Optionally, the mesh is covered by a biodegradable material.

In an exemplary embodiment of the invention, the mesh is included inside said device.

Optionally, the material comprises a fibrosis promoting substance.

In an exemplary embodiment of the invention, the device is configured to cushion between the small bones.

In an exemplary embodiment of the invention, the device comprises an annular shaped portion.

In an exemplary embodiment of the invention, said first smooth surface is located on a proximal side of said device.

In an exemplary embodiment of the invention, the device comprises a distal side with a second smoothed surface on which a second small bone may slide. Optionally, said second small bone is a trapezium.

In an exemplary embodiment of the invention, said device is designed to rupture within a time period of 1 to 30 weeks following expansion. Optionally, said device is designed to rupture within a time period of 6 to 10 weeks following expansion.

In an exemplary embodiment of the invention, said device is designed to completely biodegrade within a time period of 3 - 30 months following expansion. Optionally, said device is designed to completely biodegrade within a time period of 6 - 12 months following expansion.

In an exemplary embodiment of the invention, the device comprises said sizing comprises a diameter within a range of a factor of 0.8 to 1.2 of a diameter of said small bone adjacent said device.

In an exemplary embodiment of the invention, said sizing comprises a thickness of within the range of a factor of 0.5 to 2 of a natural distance between said small bones.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A and 1B schematically illustrate an exemplary view of the bones in the hand and an enlarged view of an area of the hand showing the thumb metacarpal and the trapezium, respectively;
FIGs. 2A - 2E schematically illustrate steps involved in replacing a CMC joint in a hand using an exemplary expandable joint implant device, according to an embodiment of the present invention;
FIGs. 3A - 3G schematically illustrate different configurations of the exemplary joint implant device, according to some embodiments of the present invention;
FIGs. 4A and 4B schematically illustrate different configurations of the exemplary joint implant device including anchoring means, according to some embodiments of the present invention;
FIG. 5 schematically illustrates another configuration of the exemplary joint implant device, according to some embodiments of the present invention;
FIG. 6 schematically illustrates a flow chart of a method for performing a CMC joint implant including the exemplary joint implant device; and
FIG. 7 schematically illustrates several views A - D of an exemplary joint implant device, according to some embodiments of the present invention.

### DESCRIPTION OF EMBODIMENTS OF THE INVENTION

The present invention, in some embodiments thereof, relates to body implantable devices and, more particularly, but not exclusively, to a small bones implant device for implanting between small bones.

An aspect of some embodiments of the present invention relates to an expandable implantable device configured to be implanted in between small bones, for inducing deposition of tissues between the bones. Throughout this disclosure, the "expandable implantable device" may be used interchangeably with "expandable joint implant device", "expandable cushioning device", "expandable spacer", "joint implant device", "expandable device", "joint device", "implant", and "device". The device may be used for partially replacing a CMC joint in a hand. Optionally, the device is used for wholly replacing the CMC joint in the hand. In some exemplary embodiments, the joint implant device may be used at other articular sites (joint areas) such as, for example, between phalanges of a hand and/or a foot, at the metatarsalphalangeal joint, between the scaphoid and the trapezoid, and at other small joints in the foot and/or the hand.

According to some embodiments, the implant is configured to substantially conform to bony surfaces in the joint area and to serve as a cushion between the bones. Optionally, the implant includes a size which matches a width of the wider bone. Optionally, the size matches a width of the narrower bone. Optionally, the implant includes a size which ranges from 50% - 90% of the width of the wider bone. Additionally or alternatively, the size ranges from 50% - 90% of the width of the narrower bone. Optionally, the implant allows relative movement of the bones while providing for tissue repair and stabilization. The implant is further configured to serve as a spacer for maintaining a separation between the bones. Optionally, length and configuration are restored to the joint substantially reducing anatomical deformation, for example, a thumb length and shape may be maintained. An advantage of using the implant over other devices known in the art is that patient rehabilitation may be initiated soon following surgical intervention, for example, within a period ranging from 1 - 14 days. Optionally, patient rehabilitation may start within a period of 1 - 3 days, 3 - 7 days, 7 - 10 days.

In some embodiments, the joint implant device may include an inflatable device such as for example, a balloon, which is inserted in a deflated state into a cavity in the joint area following a partial or complete resection of a joint surface. Once in position, the balloon may be inflated to an expanded state with a liquid, a gas, or a gel, introduced into the balloon through an inflation port. Optionally, the inflation port is located inside the balloon when in an expanded state so as to substantially prevent injury or irritation from rubbing against edges of the port. Optionally, inflation is done by temporarily attaching an inflation cannula or inflation needle to the inflation port and removing the cannula or needle when the balloon is expanded to a required size. Additionally or alternatively, the inflation port is biodegradable. Optionally, the inflation port includes a one-way valve which may be biodegradable.

In some embodiments of the present invention, an expanded size of the device may be, for example, 22mm x 20mm x 12mm. Optionally, an expanded size may be 20mm x 12mm x 8mm. Additionally or alternatively, the expanded size of the device may be such that a length of the device does not exceed 30mm, a width does not exceed 30mm, and a height does not exceed 20 mm. An expanded shape of the device may include any polyhedral shape, such as for example, rectangular, trapezoidal, cylindrical, octagonal. Optionally, the expanded shape may include a spherical shape or any curved variations of a spherical shape such as, for example, ellipsoid. A non-expanded size of the device may not exceed a length of 30mm, a width of 30mm, and a height of not more than 10mm (for example, 6mm, 4mm, 3mm or less). A wall thickness of the device may range from less than 1mm to 5mm, for example 1mm, 2mm, 3mm. A modulus of elasticity of the device may be equivalent to that of a cartilage or a tendon. Optionally, the modulus of elasticity is in a range of 30% - 150% of that of a cartilage, or a tendon. Inserting the balloon in a deflated state allows for surgical intervention involving joint arthroscopy or arthroscopic CMC joint arthroplasty, the balloon inserted through a relatively small perforation in the articular capsule. Optionally, the surgical intervention may include open surgery. Additionally or alternatively, the surgical intervention may include a full Trapezectomy or a partial Trapezectomy. Optionally, the implant may include a sponge-like device or other type of device which may be compressed for insertion and expands once positioned inside the cavity.

In some embodiments, the device may include a biodegradable material such as, for example, PLA, PLGA, polycaprolactone, polydiaxone, or other biocompatible biodegradable material, or any combination thereof, suitable for lasting a period of time in a range from 1 week to 1 year, optionally from 1 month to 4 months, optionally from 6 weeks to 12 weeks, for example 8 weeks. Additionally or alternatively, the device may include a biodegradable material such that the device ruptures within a period of 5 - 15 weeks from implantation, for example 7 - 8 weeks, the ruptured device biodegrading within a period of time ranging from 6 - 18 months from implantation, for example 9 - 14 months. Optionally, the device may include a non-biodegradable, biocompatible material such as, for example, polyethylene, polyurethane, silicon, other polymeric or non-polymeric biocompatible materials, or any combination thereof. Optionally, the device may be seamless thereby allowing an improved homogeneity in shape and/or degradation, improved structural durability to inner and/or outer stresses. More details about exemplary degradable materials and/or balloons, and ways of producing thereof, are described in US Publication No. 2008/0033471 titled "DEVICE SYSTEM AND METHOD FOR TISSUE DISPLACEMENT OR SEPARATION"

In some embodiments, the device may include a cylindrical shape and may include one or more openings (passages) extending from a first side of the device to a second opposing side of the device, for allowing fibrotic development (fibrosis) between the bones from both sides of the device (from the side of the trapezoid and the side of the metacarpal). Optionally, the passages may angularly extend from the first side to the second side. Optionally, the passages are positioned and oriented so as to achieve a desired pattern of fibrotic growth. Optionally, the passages may cover an area ranging from 10% - 50% of the total area of the first side and the second side. Creating fibrotic bridges between the bones, additionally to filling the joint area and strengthening the surrounding articular capsule, may serve to permit a full range of motion and to prevent bone shortening, for example, thumb shortening. Optionally, the device may include an internal cylindrical shape forming two concentric rings (one rings inside the other). Optionally, the external ring biodegrades before the internal ring. Optionally, the device may be star-shaped so that radial extensions may assist in promoting fibrosis. Optionally, the device may include any other shape suitable for promoting fibrosis while allowing the device to serve as a cushion and/or spacer. Optionally, an exterior of the device may be partially or wholly covered with a mesh for promoting fibrosis, in some cases the mesh covering those areas in contact with bone and which do not interfere with movement of the bones. Optionally, such a mesh is distributed to achieve a desired pattern of fibrotic growth. Optionally, such a mesh may be covered by a biodegradable material so as to not interfere with movement of the bones while the capsule is being strengthened and only then being exposed to promote fibrosis between the bones. Additionally or alternatively, an interior of the device may include such a mesh, and may protrude through a surface of the device as the device biodegrades. Optionally, the surface of the device may be coated with a slow-release substance that promotes fibrosis such as, for example, FGF. Optionally, walls of passages are coated with the substance which promotes fibrotic development. Additionally or alternatively, walls of passages are coated with a substance which substantially inhibits fibrotic development. Optionally, the surface may be coated with an anti-inflammatory substance such as, for example, steroids and or antibiotics. Optionally, the device may include seams to promote fibrosis. Optionally, the device may be filled with a fibrosis promoting agent that may be dispersed into the surrounding when the device ruptures and/or at least partially degrades.

In some embodiments, the opposing first and second sides of the device may include at least one smooth surface for allowing movement of the bones relative to the device, including sliding along the surface. For example, the first (thumb) metacarpal may move along the surface of the first side (distal side) of the device, and the device may move with respect to the trapezium along the surface of its opposing side (proximal side). Optionally, the surface may be coated with a substance which substantially prevents bone adherence. Optionally, such a substance enhances sliding of a bone on the surface. Optionally, the surface may include a substance which substantially prevents sliding of a bone over the surface.

In some embodiments of the present invention, the device may include a shape resembling a "dumbbell" including two expanded ends joined together by a connecting portion so that movement in the joint area is transmitted to each end of the device while substantially maintaining movement between the bones and the surfaces of the device to a minimum (reducing wear on the bones and possible pain). In this case, at least one of the surfaces in contact with a bone is coarse or rough thereby minimizing a relative movement of the surface with respect to the bone and allowing a transfer of movement to the connecting portion.

In some embodiments of the present invention, the device may include anchoring means (anchor) for substantially preventing dislodgement of the device during movements within the articulation. The anchor may be located on a palmar side of the device and may be attached to the articulate capsule. Optionally, the anchor may be positioned on the device at an angle not less than 30 degrees from the inflation port. Optionally, the anchor may be arrow-shaped and is configured to pierce into the palmar capsule where it may be embedded. Optionally, the anchor may include a hook for securing the attachment to the capsule or to bone. Optionally, the anchor may be adhered to the bone or to the articular capsule by means of an adhesive. Optionally, the adhesive is biodegradable. Additionally or alternatively, the anchor may be inflatable and is inflated together with the rest of the device via the inflation port. Optionally, the anchor includes a separate inflation port and is inflated following attachment to the capsule (or the bone). Optionally, a same inflation port includes separate conduits for inflating the device and the anchor. Optionally, the anchor is an integral extension of the device and is of a same material as the device. Optionally, the anchor is a separate extension of the device, and may or may not, be of a same material as the device.

Described is a method for implanting an expandable device in between at least two small bones using minimal invasive surgery such as, for example, arthroscopic intervention. The method includes perforating a small hole in an articular capsule; removing bone tissue from part of the at least two small bones to form a cavity; inserting the device in a non-expanded state into the cavity and expanding the device to a size where the device may serve both as a cushion and a spacer in between the at least two bones; and starting patient rehabilitation after a period ranging from 1 - 14 days. Optionally, patient rehabilitation may start within a period of 1 - 3 days, 3 - 7 days, 7 - 10 days.

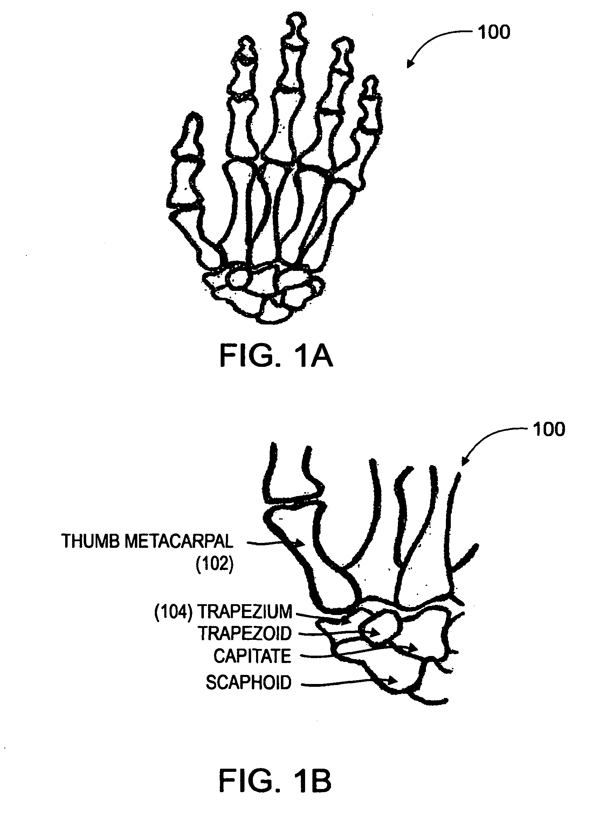

For purposes of better understanding some embodiments of the present invention, reference is first made to FIGURES 1A and 1B which schematically illustrate an exemplary view of the bones in the hand **100** and an enlarged view of an area of the hand showing the thumb metacarpal **102** and the trapezium **104**, respectively.

Before explaining at least one embodiment of the invention in detail, it is to be understood that the invention is not necessarily limited in its application to the details of construction and the arrangement of the components set forth in the following description and/or illustrated in the drawings. The invention is capable of other embodiments or of being practiced or carried out in various ways.

Before explaining at least one embodiment of the invention in detail, it is to be understood that the invention is not necessarily limited in its application to the details set forth in the following description. The invention is capable of other embodiments or of being practiced or carried out in various ways.

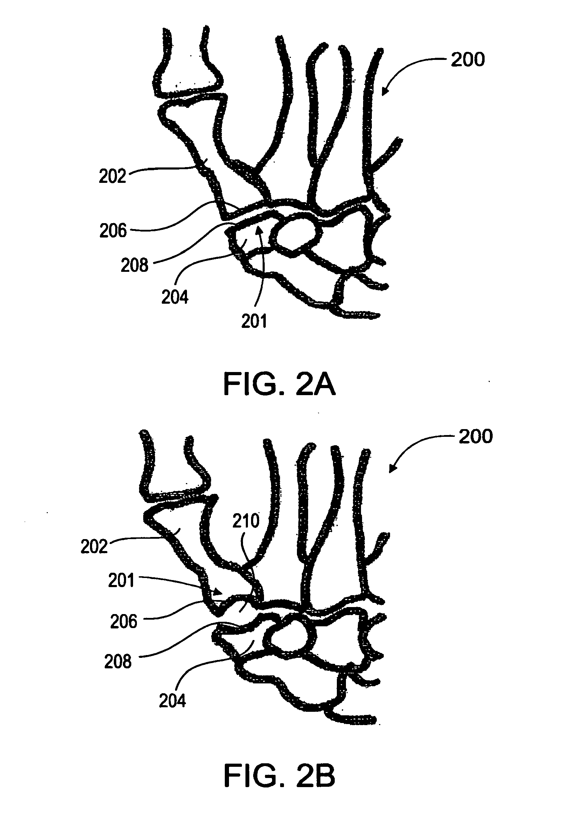

Referring now to the drawings, FIGURES 2A - 2E schematically illustrate steps included in treating a CMC joint in a hand **200** using an exemplary expandable joint spacer implant device **212**, according to an embodiment of the present invention. Optionally, the treated joint is in another articular site, for example, elsewhere in hand **200** or in a foot (not shown).

Figure 2A shows a portion of base **206** of thumb metacarpal **202** and a portion of inferior surface **208** of trapezium **204** cut so as to facilitate access to a joint area **201**.

Figure 2B shows a cavity **210** formed in joint area **201** by cutting portions of base **206** and inferior surface **208**, the cavity configured to receive implant **212**.

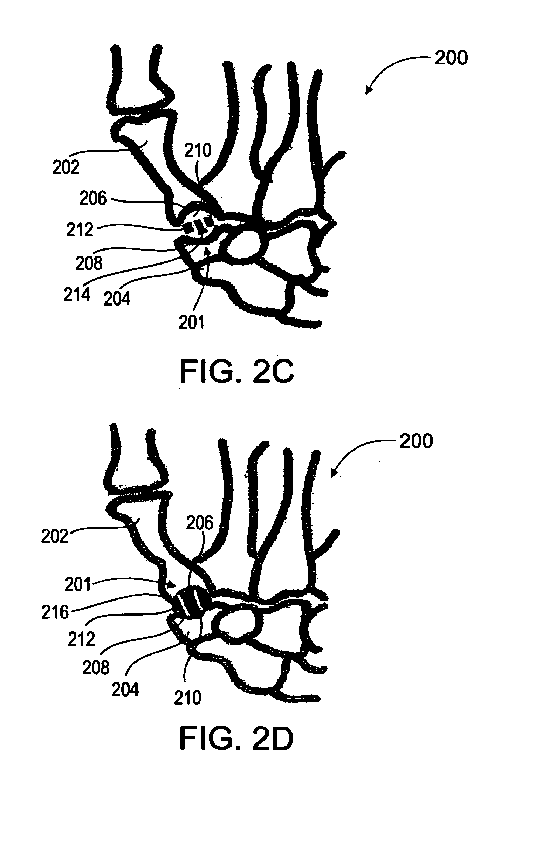

Figure 2C shows implant **212** in a non-expanded state and positioned inside cavity **210** in joint area **201**, between base **206** and inferior surface **208**. Optionally, implant **212** is in a compressed state or in a deflated state.

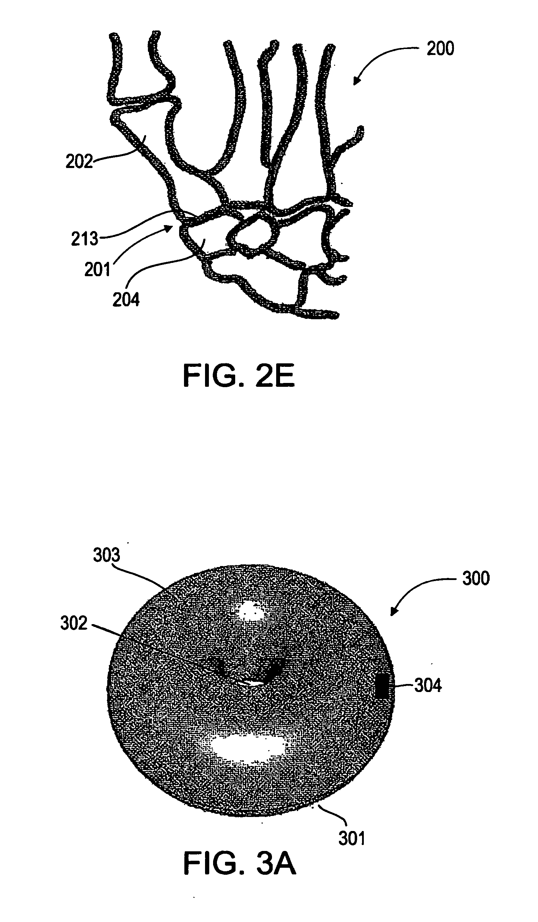

Implant **212** may include a balloon or similar inflatable device; a sponge-like device; or any other expandable device suitable to be inserted in a compressed state, or a deflated state into cavity **210** through a relatively small perforation in the articular capsule. Implant **212** may include a biocompatible and/or a biodegradable material. Biocompatible materials may include, for example, polyethylene, polyurethane, silicon, or other biocompatible polymeric or non-polymeric materials, or any combination thereof. Biodegradable materials may include, for example, PLA, PLGA, polycaprolactone, polydiaxone or other biodegradable material, or any combination thereof, suitable for degrading within a period of any predetermined window of time, optionally between 6 - 12 weeks from insertion into cavity **210**, for example, optionally within 8 weeks. Optionally, device **212** may be similar to those devices shown in Figures 3A - 5 described further on below.

Implant **212** may include an inflation port **214** through which an expansion fluid, which may be a liquid, a gas, or a gel, may be introduced to inflate the device. The expansion fluid may be a biocompatible fluid, and/or a biodegradable fluid. For example, the liquid may be a 0.9% saline, a Ringer solution or a Hartman solution. The gel may be, for example, an absorbable haemostatic agent such as gelatin, cellulose, or bovine collagen, or a biodegradable synthetic adhesive such as poly ethylene glycol (PEG). The gas may be for example, oxygen, nitrogen, or any other gas readily absorbed by the human body, or any combination thereof.

Figure 2D shows implant **212** is in an expanded (inflated) state, following introducing of the expansion fluid into the device through inflation port **214**. Inflation port **214** is not visible as the port is inside device **212** following inflation of the device.

Inflation of implant **212** is done by temporarily attaching an inflation cannula, optionally an inflation needle, to inflation port **214**, and injecting the expansion fluid into the device. Once device **212** is expanded to the appropriated size, inflation port **214** is sealed and the inflation cannula removed.

Once expanded to the appropriate size, device **212** optionally fills cavity **210** and optionally conforms to the shape of the cavity, including the reshaped portions of base **206** and inferior surface **208**. In the expanded state, implant **212** serves as a cushion and/or a spacer between thumb metacarpal **202** and trapezium **204**. Optionally, device **212** includes holes for promoting fibrotic development between thumb metacarpal **202** and trapezium **204**. Optionally, a contour of device **212** is suitable for promoting fibrotic development, for example by including extensions such as radial extensions in a starred shape. Additionally or alternatively, device **212** may include a mesh for promoting fibrotic development or may be coated with a fibrosis promoting substance such as, for example, FGF. Optionally, device **212** may include a coating of an anti-inflammatory substance such as, for example, steroids and/or antibiotics. Optionally, device **212** may include a seam, which may optionally assist to promote fibrosis. Optionally, device **212** may be seamless.

In the expanded state, device **212** may be configured to allow thumb metacarpal **202** to slide along a distal side of the device abutting with base **206**, and for the device to slide relative to trapezium **204** along a proximal side of the device abutting inferior surface **208**. Optionally, device **212** slides relative to thumb metacarpal **202** and/or to trapezium **204**.

Device **212** may optionally include an anchor **216** for substantially preventing possible dislodgement of the device from within cavity **210** when expanded. Anchor **216** may be attached to the articular capsule or to a bone, for example, to the scaphoid below the trapezium or to the trapezoid next to the trapezium.

Figure 2E shows joint area 201 following biodegradation of implant **212** and a fibrotic bridge **213** formed between thumb metacarpal and trapezium **204** (synfibrosis). Fibrotic bridge **213** filled joint area **201** and strengthened the surrounding articular capsule. Due to an optional continuous relative movement of metacarpal **202** and trapezium **204** therebetween and with respect to implant **212**, a complete bone fusion is substantially prevented and a synfibrosis, a synarthrosis or a synostosis is optionally formed in the space previously occupied by implant **212**, allowing at least partial movement of thumb metacarpal **202** with respect to trapezium **204**.

Reference is now also made to FIGURES 3A - 3G which schematically illustrate different, non-limiting, configurations of an exemplary joint implant device, according to some embodiments of the present invention. The implants shown at **300**, **310**, **320**, **330**, **340**, and **350** may be similar to that shown in FIGURES 2C and 2D at **212**. The embodiments shown are not intended to be limiting in any form, and it should be evident to an ordinary person skilled in the art that many other configurations (including device shapes, hole shapes, extensions, etc.) may be used.

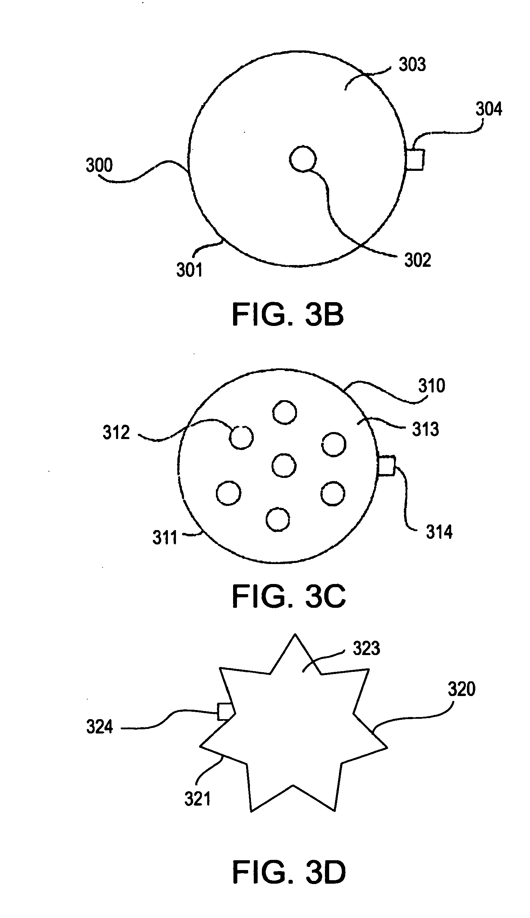

FIGURES 3A and 3B show a perspective view of implant **300** and a layout view of the implant. Implant **300** includes a torus (optionally cylindrical) shape with an opening **302** (passage) extending through the device ("donut-shaped") for promoting fibrotic development through the opening from a direction from both thumb metacarpal **202** and trapezium **204**. Optionally, opening **302** which is shown with a circular shape may include other shapes, for example rectangular, triangular, star-shaped, an 8 - shape, or other polygonal shapes. Optionally, proximal side **301** and distal side **303** may include a coating of a slow-release substance for promoting fibrosis, and/or with an anti-inflammatory substance such as, for example, a steroid or an antibiotic. Optionally, proximal side **301** and/or distal side **303** may be treated with a substance to prevent bone adhesion. Additionally or alternatively, the substance may enhance bone movement along the surfaces. Implant **300** is inserted into cavity **210** in a deflated mode and is positioned so that thumb metacarpal **202** abuts distal side **303** of the device when expanded and trapezium **204** abuts proximal side **301**. Both distal side **303** and proximal side **301** include a smooth surface for allowing relative movement of thumb metacarpal **202** and trapezium **204** with respect to device **300**. Implant **300** includes an inflation port **304** to which an inflation cannula or needle may be attached for introducing expansion fluid into the device.

FIGURE 3C shows a layout view of implant **310**. Implant **310** includes a cylindrical shape with a plurality of openings **312** (passages) extending through the device for promoting fibrotic development through the openings from a direction from both thumb metacarpal **202** and trapezium **204**. Optionally, opening **312** which is shown with a circular shape may include other shapes, for example rectangular, triangular, star-shaped, an 8-shape, or other polygonal shape, or any combination thereof. Optionally, proximal side **311** and distal side **313** may include a coating of a slow-release substance for promoting fibrosis, and/or with an anti-inflammatory substance such as, for example, a steroid or an antibiotic. Optionally, proximal side **311** and/or distal side **313** may be treated with a substance to prevent bone adhesion. Additionally or alternatively, the substance may enhance bone movement along the surfaces Implant **310** is inserted into cavity **210** in a deflated mode and is positioned so that thumb metacarpal **202** abuts distal side **313** of the device when expanded and trapezium **204** abuts proximal side **311**. Both distal side **313** and proximal side **311** include a smooth surface for allowing relative movement of thumb metacarpal **202** and trapezium **204** with respect to device **310.** Implant **310** includes an inflation port **314** to which an inflation cannula or needle may be attached for introducing expansion fluid into the device.

FIGURE 3D shows a layout view of implant **320**. Implant **320** includes a starred shape including radial extensions for promoting fibrotic development through the extensions from both thumb metacarpal **202** and trapezium **204**. Optionally, proximal side **321** and distal side **323** may include a coating of a slow-release substance for promoting fibrosis, and/or with an anti-inflammatory substance such as, for example, a steroid or an antibiotic. Optionally, proximal side **321** and/or distal side **323** may be treated with a substance to prevent bone adhesion. Additionally or alternatively, the substance may enhance bone movement along the surfaces. Optionally, implant **320** may include one or more passages extending directly or angularly from proximal side **321** to distal side **323**. Implant **320** is inserted into cavity **210** in a deflated mode and is positioned so that thumb metacarpal **202** abuts distal side **323** of the device when expanded and trapezium **204** abuts proximal side **321**. Both distal side **323** and proximal side **321** include a smooth surface for allowing relative movement of thumb metacarpal **202** and trapezium **204** with respect to device **320**. Implant **320** includes an inflation port **324** to which an inflation cannula or needle may be attached for introducing expansion fluid into the device.

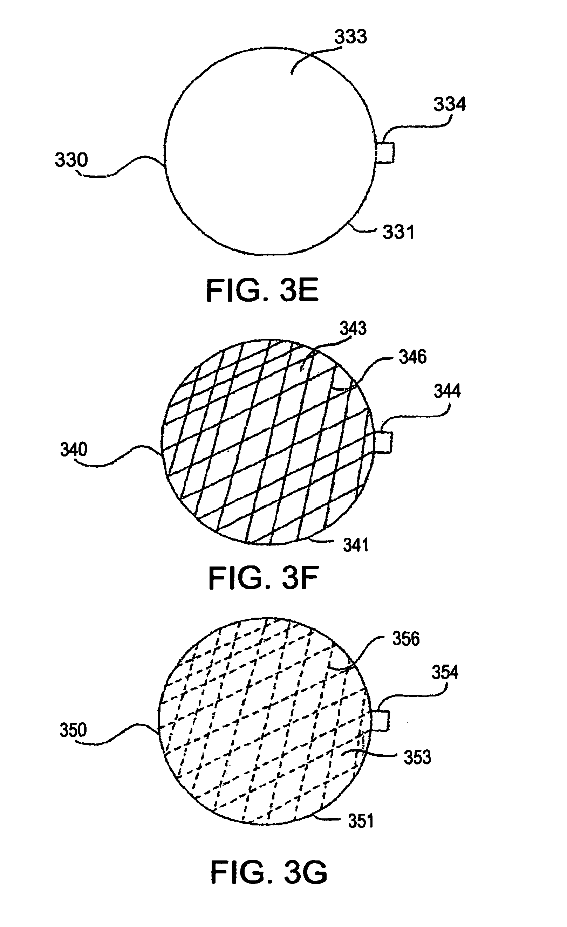

FIGURE 3E shows a layout view of implant **330** which includes a cylindrical shape. Implant **330** is inserted into cavity **210** in a deflated mode and is positioned so that thumb metacarpal **202** abuts a distal side **333** of the device when expanded and trapezium **204** abuts a proximal side **331**. Both distal side **333** and proximal side **331** include a smooth surface for allowing relative movement of thumb metacarpal **202** and trapezium **204** with respect to device **330**. Optionally, proximal side **331** and distal side **331** may include a coating of a slow-release substance for promoting fibrosis, and/or with an anti-inflammatory substance such as, for example, a steroid or an antibiotic. Optionally, proximal side **331** and/or distal side **333** may be treated with a substance to prevent bone adhesion. Additionally or alternatively, the substance may enhance bone movement along the surfaces. Implant **330** includes an inflation port **334** to which an inflation cannula or needle may be attached for introducing expansion fluid into the device.

FIGURE 3F shows a layout view of implant **340.** Implant **340** includes a mesh **346** covering a portion, optionally a whole, of a proximal side **341** and/or a distal side **343** of the implant for promoting fibrotic development from both thumb metacarpal **202** and trapezium **204**. Optionally, only portions of proximal side **341** and/or distal side **343** not in contact with moving bones are covered by the mesh. Additionally or alternatively, mesh **346** is covered by a biodegradable material which exposes the mesh only after a period of time during which the articular capsule is strengthened, for promoting fibrosis between thumb metacarpal **202** and trapezium **204**. The material provides both distal side **343** and proximal side **341** with a smooth surface for allowing relative movement of thumb metacarpal **202** and trapezium **204** with respect to device **340**. Optionally, the material prevents bone adhesion. Optionally, proximal side **341** and distal side **343** may include a coating of a slow-release substance for promoting fibrosis, and/or with an anti-inflammatory substance such as, for example, a steroid or an antibiotic. Implant **340** is inserted into cavity **210** in a deflated mode and is positioned so that thumb metacarpal **202** abuts distal side **343** of the device when expanded and trapezium **204** abuts proximal side **341**. Implant **340** includes an inflation port **344** to which an inflation cannula or needle may be attached for introducing expansion fluid into the device.

FIGURE 3G shows a layout view of implant device **350** including a mesh **356** inside the device for promoting fibrotic development from both thumb metacarpal **202** and trapezium **204**. Device **350** may be similar to device **340** with the exception that mesh **356** is internally located in the expandable portion of the device, and protrudes through a surface of the device as the device biodegrades. Optionally, mesh **356** is exposed once a major portion of device **350**, or optionally a whole of the device, biodegrades.

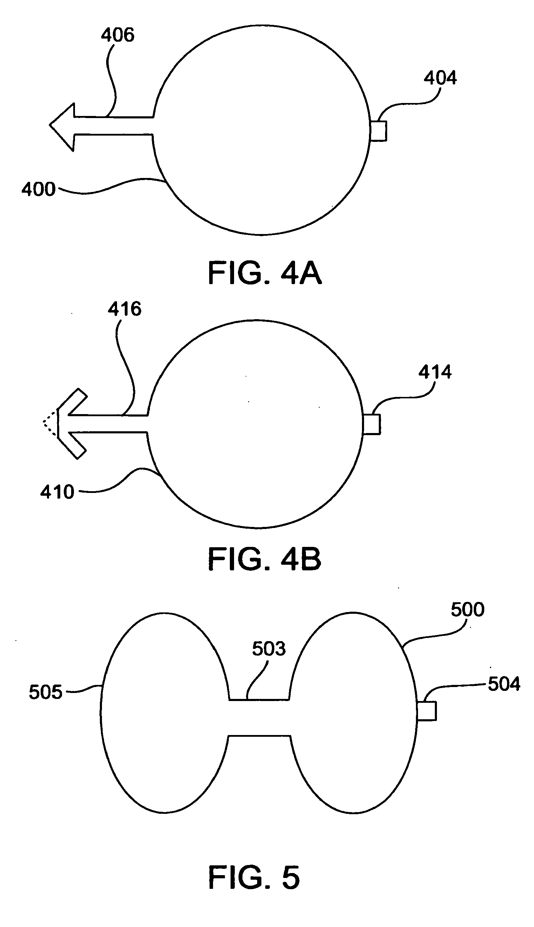

Reference is now also made to FIGURES 4A and 4B which schematically illustrate different, non-limiting configurations of the exemplary joint implant device including anchoring means, according to some embodiments of the present invention. The implants, shown at **400** and **410**, may be similar to that shown in FIGURES 2C and 2D at **212**, or FIGURES 3A - 3G at **300**, **310**, **320**, **330**, **340**, or **350**, respectively, with a difference that implants **400** and **410** include an anchor **406** and **416** respectively. The embodiments shown are not intended to be limiting in any form, and it should be evident to an ordinary person skilled in the art that many other configurations (including device shapes, hole shapes, extensions, etc.) may be used.

Anchor **406** in FIGURE 4A and anchor **416** in FIGURE 4B are configured to substantially prevent dislodgement of device **400** and **410** respectively, during movements within cavity **210**. Anchors **406** and **416** may be located on a palmar side of device **400** and **410**, respectively, and may be attached to the articulate capsule. Optionally, as shown in FIGURE 4A, anchor **406** may be arrow-shaped and is configured to pierce into the palmar capsule where it may be embedded. Optionally, as shown in FIGURE 4B, anchor **416** may include a hook for securing the attachment to the capsule or to bone. Optionally, anchor **416** may include an arrow-shaped head to facilitate piercing of the capsule prior to attachment of the hooks. Additionally or alternatively, anchor 406 and/or 416 is attached to the articular capsule or the bone by means of a biodegradable adhesive.

According to some embodiments, anchors **406** and/or **416** may be inflatable and may be inflated together with the expansive portion (inflatable portion) of device **400** and **410** via inflation ports **404** and **414**, respectively. Optionally, anchors **406** and/or **416** may include separate inflation ports and may be inflated following attachment to the capsule (or the bone). Optionally, inflation ports **404** and/or **414** include separate conduits for inflating the device and the anchor. Optionally, anchors **406** and/or **416** may be an integral extension of device **400** and/or **410,** respectively, and of a same material as the device. Optionally, **406** and/or **416** may be a separate extension of device **400** and/or **410**, respectively, and may or may not be of a same material as the device. Optionally, anchors **406** and/or **416** are made of a biocompatible and/or biodegradable material.

Reference is now also made to FIGURE 5 which schematically illustrates another configuration of an exemplary joint implant device **500**, according to some embodiments of the present invention. Implant **500** may be similar to that shown in FIGURES 2C and 2D at **212**, or FIGURES 3A - 3G at **300**, **310**, **320**, **330**, **340**, or **350**, respectively, or FIGURES 4A or 4B at **400** or **410**, respectively with a difference that implant **500** includes a "dumbbell" shape.

Implant **500**, when expanded, includes two relatively large expanded end sections **505** joined together by a relatively narrower connecting portion **503**. Inflation of end sections **505** and connecting portion **503** is by means of an expansion fluid introduced through inflation port **504**, as previously described for other embodiments. Optionally, connecting portion **503** is a non-inflatable element of a suitable size to be inserted into cavity **210** with end sections **505** deflated. Implant **500** is configured to substantially restrain movement of thumb metacarpal **202** and trapezium **204** relative to the implant. Movement between the two bones is through connecting portion **503**, which acts as a joint, with movement of each bone transmitted to end sections **505** which act as supports.

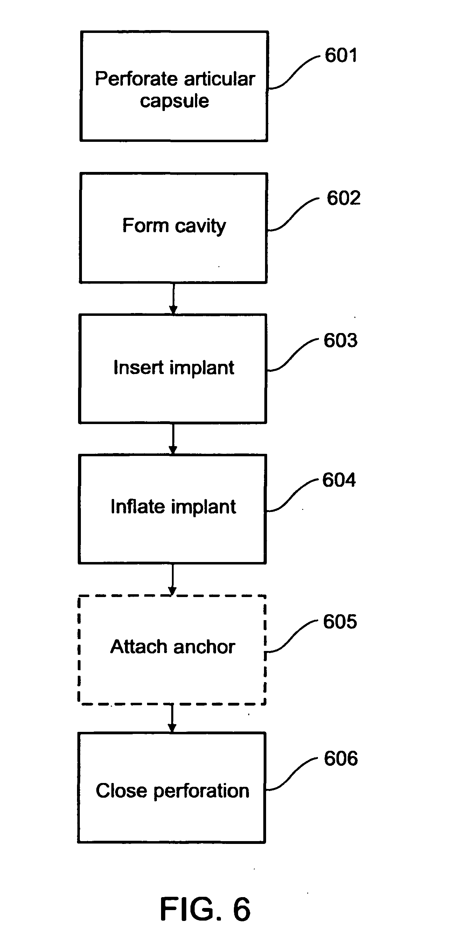

Reference is now made to FIGURE 6 which schematically illustrates a flow chart of a method for performing a CMC joint implant including exemplary joint implant device **212**. Optionally, any of the devices **300**, **310**, **320**, **330**, **340**, **350**, **400**, **410**, or **500** may be used for implementing the method. The method described below is not intended to be limiting in any way and it should be evident to an ordinary person skilled in the art that there may be many other ways of implementing the method, including, for example, using different steps, a different order of the steps, skipping steps, inserting steps.

Optionally at **601**, a partial trapezectomy is to be performed using arthroscopy. A hole is perforated in the articular capsule and a portion of base **206** in thumb metacarpal **202** is cut. A portion of inferior surface **208** in trapezium **204** is also cut, both cuts to facilitate access to joint area **201** to prepare cavity **210**. Optionally, a full trapezectomy may be performed wherein trapezium **204** is completely removed. Optionally, the partial trapezectomy may be removed using open surgery.

Optionally at **602**, base **206** and inferior surface **208** are further cut and shaped to form cavity **210** in joint area **201**. Cavity **210** is of a size to allow non-expanded device **212** to be inserted into the cavity and positioned such that, when expanded, will act as a cushion and/or spacer between thumb metacarpal **202** and trapezium **204**. Optionally, expanded device **212** is secured within cavity **210** any may not be dislodged through the hole in the articular cavity. Cavity **210** shall also allow for device **212** to be positioned such that there is relatively easy access to inflation port **214** for expanding the device and for sealing the inflation port once the device is expanded.

Optionally at **603**, implant **212** is inserted into cavity **210**. An inflation cannula which may include an inflation needle for connecting to inflation port **214** may be configured to clasp device **212** at a distal end, for positioning the device inside cavity **210**. Optionally, device **212** may be rolled on a distal end of the inflation cannula in a deflated state for inserting in cavity **210**. Optionally, other methods known in the art for placing implants using arthroscopy may be used.

Optionally at **604**, an expansion fluid is injected through the inflation cannula into inflation port **214**. The expansion fluid may include a liquid, a gas, or a gel, or any combination thereof. Device **212** is expanded to cover a portion, optionally a whole, of cavity **210** forming a cushion and/or spacer between thumb metacarpal **202** and trapezium **204**. Device **212** partially, optionally wholly, conforms to a shape of the bone surfaces of base **206** and inferior surface **208.** Once expanded to the desired length, inflation port **214** may be sealed. Optionally, inflation cannula may be detached from device **212** and extracted from the articular capsule. Optionally, the inflation cannula is not extracted for possible use in attaching anchor **216** and/or for performing other arthroscopic operations.

Optionally at **605**, anchor **216** may be attached to the articulate capsule. Optionally, anchor **216** may be attached to a bone, for example, to the scaphoid located below the trapezium or to the trapezoid located next to the trapezium.

Optionally at **606**, the perforation in the articular capsule is closed and the arthroscopic procedure is finalized. Patient may start rehabilitation after a period ranging from 1 - 14 days. Optionally, patient rehabilitation may start within a period of 1 - 3 days, 3 - 7 days, 7 - 10 days. Optionally, device **212** may include a biodegradable material such that the device ruptures within a period of 5 - 15 weeks from implantation, for example 7 - 8 weeks. During this time the articular capsule is strengthened and limited fibrotic development occurs around and through the device. Optionally, the ruptured device biodegrades within a period of time ranging from 6 - 18 months from implantation, for example 9 - 14 months, during which time substantially complete fibrotic development occurs.

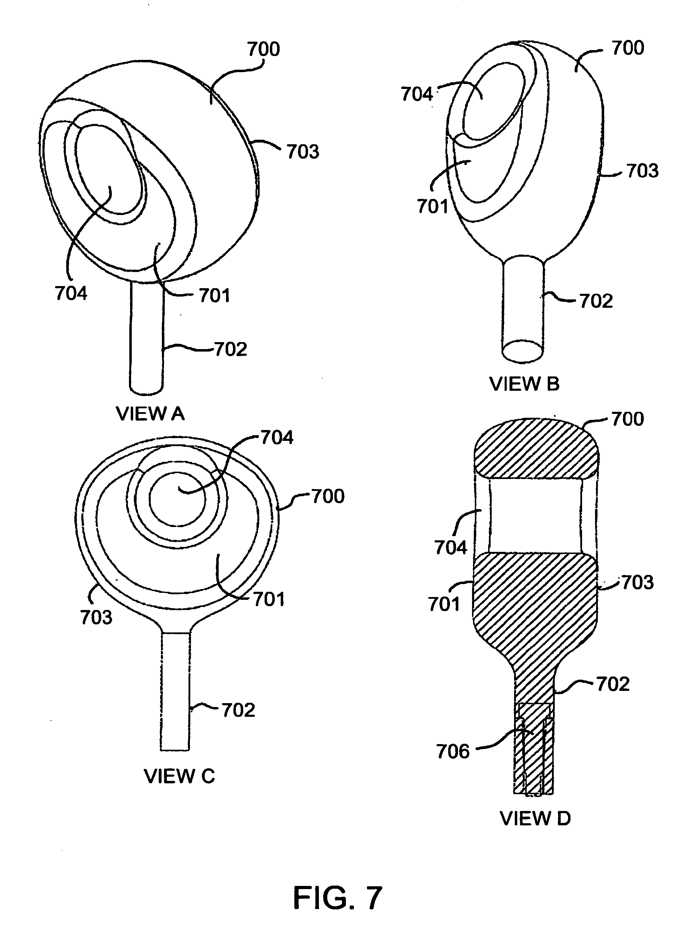

Reference is now also made to FIGURE 7 which schematically illustrates several views A - D of an exemplary joint implant device **700**, according to some embodiments of the present invention. Implant **700** may be similar to that shown in FIGURES 2C and 2D at **212**, or FIGURES 3A - 3G at **300**, **310**, **320**, **330**, **340**, or **350**, respectively, or FIGURES 4A or 4B at **400** or **410**, respectively. The exemplary embodiment shown herein is not intended to be limiting in any form, and it should be evident to an ordinary person skilled in the art that many other configurations (including device shapes, hole (passage) shapes, number of passages , etc.) may be used for inplant **700.**

Views A and B show a perspective view of implant **700**, view C shows a layout view of the implant, and view D shows a sectional view of the implant, according to an exemplary embodiment. Implant **700** which may include a shape as shown includes an opening **704** (passage) extending through the device for promoting fibrotic development through the opening from a direction from both thumb metacarpal **202** and trapezium **204**. Optionally, passage **704** which is shown with a circular shape may include other shapes, for example rectangular, triangular, star-shaped, an 8 - shape, or other polygonal shapes. Passage **704** is optionally distally positioned from a one-way inflation valve **706** included inside an inflation port **702** substantially limiting possible damage to the device due to the insertion pressure of an expansion fluid. An inflation cannula and/or needle may be attached to inflation port **702** and inflation valve **706** for introducing the expansion fluid into the device. Optionally, inflation port **702** includes a biodegradable material. Optionally, inflation valve includes a biodegradable material.

According to some embodiments, proximal side **701** and distal side **703** may include a coating of a slow-release substance for promoting fibrosis, and/or with an anti-inflammatory substance such as, for example, a steroid or an antibiotic. Optionally, proximal side **701** and/or distal side **703** may be treated with a substance to prevent bone adhesion. Additionally or alternatively, the substance may enhance bone movement along the surfaces. Implant **700** is inserted into cavity **210** in a deflated mode and is positioned so that thumb metacarpal **202** abuts distal side **703** of the device when expanded and trapezium **704** abuts proximal side **701**. Both distal side **703** and proximal side **701** include a smooth surface for allowing relative movement of thumb metacarpal **202** and trapezium **204** with respect to device **700**

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## Claims

1. A soft, expandable, implantable device (212) sized for spacing between small bones **characterised in that** it comprises a first smooth surface on which a first small bone may slide.

2. A device according to claim 1, wherein said device is biodegradable in the body.

3. The device of claim 1 or claim 2 wherein said small bones comprise bones of a human hand or foot.

4. The device of claim 3 wherein said small bones comprise at least one of a trapezium, a trapezoid bone, a metacarpal bone, and a scaphoid bone.

5. The device of any of the preceding claims comprising an inflation port for inflating said device.

6. The device of any of the preceding claims comprising one or both of at least one passage extending from a distal side to a proximal side of the device, and at least one passage extending from a first lateral side to an opposite second lateral side of the device for promoting fibrotic development between said small bones.

7. The device of claim 6, wherein at least one of said at least one passage includes has a diameter that is at feast 20% of a maximal extent of the device.

8. The device of any of the preceding claims comprising a mesh for promoting fibrotic development between said small bones.

9. The device of any of the preceding claims configured to cushion between the small bones.

10. The device of any of the preceding claims comprising an annular shaped portion.

11. The device of any of the preceding claims, wherein said first smooth surface is located on a proximal side of said device and said device includes a distal side with a second smoothed surface on which a second small bone may slide.

12. The device of any of the preceding claims wherein said device is designed to rupture within a time period of 1 to 30 weeks following expansion.

13. The device of any of the claims 1-11, wherein said device is designed to completely biodegrade within a time period of 3 - 30 months following expansion.

14. The device of any of the preceding claims wherein said sizing comprises a diameter within a range of a factor of 0.8 to 1.2 of a diameter of said small bone adjacent said device.

15. The device of any of the preceding claims wherein said sizing comprises a thickness of within the range of a factor of 0.5 to 2 of a natural distance between said small bones.

## Patentansprüche

1. Weiches, ausdehnbares, implantierbares Gerät, das eine Größe hat, die eine Abstandhaltung zwischen kleinen Knochen ermöglicht, wobei es dadurch charakterisiert ist, dass es eine erste glatte Oberfläche umfasst, auf der ein erster kleiner Knochen gleiten kann.

2. Gerät gemäß Anspruch 1, wobei das Gerät im Körper biologisch abbaubar ist.

3. Gerät gemäß Anspruch 1 oder Anspruch 2, wobei die kleinen Knochen die Knochen einer menschlichen Hand oder eines menschlichen Fußes umfassen.

4. Gerät gemäß Anspruch 3, wobei die kleinen Knochen mindestens eines aus einem großen Vieleckbein, einem kleinen Vieleckbein, einem Mittelhandknochen und einem Kahnbein umfassen.

5. Gerät gemäß einem beliebigen der vorangegangenen Ansprüche, einen Aufblasanschluss zum Aufblasen des Gerätes umfassend.

6. Gerät gemäß einem beliebigen der Vorangegangenen Ansprüche, eines oder beide aus mindestens einem Durchgang, welcher sich von einer distalen Seite zu einer proximalen Seite des Geräts hin erstreckt, und mindestens einem Durchgang, welcher sich von einer ersten lateralen Seite zu einer gegenüberliegenden zweiten lateralen Seite des Geräts hin erstreckt, umfasst, wodurch die fibrotische Entwicklung zwischen den kleinen Knochen gefördert wird.

7. Gerät gemäß Anspruch 6, wobei mindestens einer des mindestens einen Durchgangs einen Durchmesser aufweist, der mindestens 20% der maximalen Ausmaße des Gerätes ausmacht.

8. Gerät gemäß einem beliebigen der vorangegangene Ansprüche, ein Netz zur Förderung der fibrotischen Entwicklung zwischen den kleinen Knochen umfassend.

9. Gerät gemäß einem beliebigen der vorangegangenen Ansprüche, wobei es so konfiguriert ist, dass es eine Polsterung zwischen den kleinen Knochen bildet.

10. Gerät gemäß einem beliebigen der vorangegangenen Ansprüche, einen ringförmigen Abschnitt umfassend.

11. Gerät gemäß einem beliebigen der vorangegangene Ansprüche, wobei die erste glatte Oberfläche sich auf einer proximalen Seite des Geräts befindet und das Gerät eine distale Seite mit einer zweiten geglätteten Oberfläche umfasst, auf der ein zweiter kleiner Knochen gleiten kann.

12. Gerät gemäß einem beliebigen der vorangegangene Ansprüche, wobei das Gerät so konzipiert ist, dass es innerhalb des Zeitraums von 1 bis 30 Wochen nach der Ausdehnung reisst.

13. Gerät gemäß einem beliebigen der Ansprüche 1 - 11, wobei das Gerät so konzipiert ist, dass es innerhalb des Zeitraums von 3 - 30 Monaten nach der Ausdehnung vollstandig biologisch abgebaut wird.

14. Gerät gemäß einem beliebigen der vorangegangenen Ansprüche, wobei seine Größe so bemessen ist, dass sie einen Durchmesser aufweist, der innerhalb eines Bereiches des Faktors 0,8 bis 1,2 des Durchmessers des kleinen Knochens liegt, welcher sich neben dem Gerät befindet.

15. Gerät gemäß einem beliebigen der vorangegangenen Ansprüche, wobei seine Größe so bemessen ist, dass sie eine Dicke aufweist, die innerhalb eines Bereiches des Faktors 0,5 bis 2 des natürlichen Abstandes zwischen den kleinen Knochen liegt.

## Revendications

1. Dispositif implantable, déployable, mou, dimensionné en vue d'un espacement entre de petits os, **caractérisé en ce qu'**il comprend une première surface lisse sur laquelle un premier petit os peut glisser.

2. Dispositif selon la revendication 1, où ledit dispositif est biodégradable dans le corps.

3. Dispositif selon la revendication 1 ou la revendication 2, dans lequel lesdits petits os comprennent des os d'une main humaine ou d'un pied humain.

4. Dispositif selon la revendication 3, dans lequel lesdits petits os comprennent au moins un os parmi un trapèze, un os trapézoïde, un os métacarpien, et un os scaphoïde.

5. Dispositif selon l'une quelconque des revendications précédentes, comprenant un orifice de gonflement en vue d'un gonflement dudit dispositif.

6. Dispositif selon l'une quelconque des revendications précédentes, comprenant un ou les deux d'au moins un passage s'étendant depuis un côté distal vers un côté proximal du dispositif, et d'au moins un passage s'étendant depuis un premier côté latéral vers un deuxième côté latéral opposé du dispositif en vue de favoriser un développement fibrotique entre lesdits petits os.

7. Dispositif selon la revendication 6, dans lequel au moins un dudit au moins un passage présente un diamètre qui est d'au moins 20 % d'une étendue maximale du dispositif.

8. Dispositif selon l'une quelconque des revendications précédentes, comprenant un maillage en vue de favoriser un développement fibrotique entre lesdits petits os.

9. Dispositif selon l'une quelconque des revendications précédentes, configuré pour réaliser un amortissement entre les petits os.

10. Dispositif selon l'une quelconque des revendications précédentes, comprenant une partie de forme annulaire.

11. Dispositif selon l'une quelconque des revendications précédentes, dans lequel ladite première surface lisse est localisée sur un côté proximal dudit dispositif et ledit dispositif comprend un côté distal doté d'une deuxième surface lissée sur laquelle un deuxième petit os peut glisser.

12. Dispositif selon l'une quelconque des revendications précédentes, où ledit dispositif est conçu pour se rompre dans un intervalle de temps de 1 à 30 semaines suivant son déploiement.

13. Dispositif selon l'une quelconque des revendications 1 à 11, où ledit dispositif est conçu pour se biodégrader complètement dans un intervalle de temps de 3 à 30 mois suivant son déploiement.

14. Dispositif selon l'une quelconque des revendications précédentes, dans lequel ledit dimensionnement comprend un diamètre dans une plage d'un facteur allant de 0,8 à 1,2 d'un diamètre dudit petit os adjacent audit dispositif.

15. Dispositif selon l'une quelconque des revendications précédentes, dans lequel ledit dimensionnement comprend une épaisseur dans la plage d'un facteur allant de 0,5 à 2 d'une distance naturelle entre lesdits petits os.
